# DEMANDE DE BREVET EUROPEEN

(11) **EP 0 755 658 A1**
(43) Date de publication de la demande: **29.01.1997**
(21) Numéro de dépôt: 96420250.1
(22) Date de dépôt: 24.07.1996
(51) Int. Cl.: A61B 17/68, A61B 17/72

(54) **Dispositif pour la réduction et maintien des fractures osseuses**

(30) Priorité: 28.07.1995 FR 9509441
(71) Demandeur: Groupe Lepine, F-69003 Lyon (FR)
(72) Inventeur: Dambreville, Alain, 29000 Quimper (FR); Pfaifer, Patrick, 69003 Lyon (FR)
(74) Mandataire: Maureau, Philippe

(57) **Abrégé**

Selon l'invention, ce dispositif comprend :
- un élément (6) en forme de grappin, comprenant une tige centrale (6a) semi-rigide, c'est-à-dire présentant une souplesse relative perpendiculairement à son axe longitudinal, apte à traverser les parties osseuses (2,2a) à maintenir, cette tige (6a) étant prolongée, à une extrémité, par au moins deux branches (6b), recourbées sur sensiblement 180 degrés, dont les extrémités libres sont acérées, et comprenant, à son autre extrémité, un anneau (6c) pour la traction de l'élément en forme de grappin (6) à travers les parties osseuses (2,2a) à maintenir ;
- une rondelle d'arrêt (8), et
- un manchon métallique (9), apte à être engagé sur la tige (6a) jusqu'à venir en appui contre la rondelle d'arrêt (9), et à être serti sur la tige (6a) après réunion des parties osseuses à maintenir et mise en tension de l'élément (6) en forme de grappin

## Description

La présente invention concerne un dispositif de maintien de parties osseuses après ostéotomie, ou de réduction d'une fracture osseuse et de maintien des parties osseuses fracturées, une fois celles-ci réunies.

Elle concerne également un instrument adapté à la mise en place de ce dispositif.

Il est bien connu de maintenir les parties d'un os ayant fait l'objet d'une ostéotomie, ou de réduire une fracture osseuse et de maintenir les parties osseuses fracturées, au moyen de pièces rigides telles que des clous, des vis ou des plaques rigides, notamment métalliques.

Dans certains cas, l'ostéotomie ou la fracture est située à proximité d'un ancrage musculaire, donc à l'emplacement d'un os soumis à des contraintes en traction importantes. Les dispositifs rigides existants sont inappropriés pour ces indications particulières, étant donné qu'il peuvent se désolidariser de l'os sous l'effet de ces contraintes.

Les différentes pièces des dispositifs existants peuvent ne pas pouvoir être mises en place en cas de dimensions réduites du site osseux, par exemple en cas d'ostéotomie ou de fracture de la rotule ou de certaines parties d'articulations, comme l'olécrane, le grand trochanter ou le plateau tibial.

Le positionnement de ces pièces rigides par rapport à l'os peut, en outre, ne pas être optimal, selon la configuration du site et l'orientation de l'ostéotomie ou de la fracture, rendant l'intervention délicate.

Dans d'autres cas, la mise en place de ces pièces pourrait avoir des conséquences dommageables, notamment occuper ou détériorer le site d'ancrage osseux d'un tendon.

La présente invention vise à remédier à ces différents inconvénients, en fournissant un dispositif pouvant être implanté dans tout type de site osseux, même à proximité d'un ancrage musculaire, en cas de site ayant des dimensions réduites ou une configuration particulière, quelle que soit l'orientation de la fracture, et sans porter atteinte à l'ancrage osseux d'un tendon pouvant se trouver à proximité.

A cette fin, le dispositif comprend :
- un élément en forme de grappin, comprenant une tige centrale semi-rigide, c'est-à-dire présentant une souplesse relative perpendiculairement à son axe longitudinal, apte à traverser les parties osseuses à maintenir, cette tige étant prolongée, à une extrémité, par au moins deux branches, recourbées sur sensiblement 180 degrés, dont les extrémités libres sont acérées, et comprenant, à son autre extrémité, un anneau pour la traction de l'élément en forme de grappin à travers les parties osseuses à maintenir ;
- une rondelle d'arrêt, destinée à être engagée sur la tige, après que celle-ci ait traversé les parties osseuses à maintenir, et à venir en appui contre l'une de ces parties osseuses, et
- un manchon métallique, apte à être engagé sur la tige jusqu'à venir en appui contre la rondelle d'arrêt, et à être serti sur la tige après réunion des parties osseuses à maintenir et mise en tension de l'élément en forme de grappin.

Pour la mise en place de ce dispositif, un trou est tout d'abord aménagé dans les parties osseuses, plus ou moins perpendiculairement aux surfaces à réunir de celles-ci, puis l'élément en forme de grappin est engagé dans ce trou jusqu'à ce que son extrémité pourvue de l'anneau débouche du côté opposé au côté d'introduction.

Les branches recourbées peuvent être orientées, par pivotement de l'élément en forme de grappin sur lui-même, de la manière la plus appropriée en fonction de la configuration spécifique du site osseux ou de la présence éventuelle d'un ancrage ligamentaire.

La rondelle d'arrêt et le manchon sont engagés sur l'extrémité de la tige dépassant de l'os jusqu'à venue de la rondelle d'arrêt en appui contre la paroi osseuse.

Une traction est alors exercée sur l'élément en forme de grappin de manière à insérer les extrémités des branches recourbées dans la paroi osseuse puis rapprocher progressivement les parties osseuses.

Une fois les parties osseuses réunies, la traction est poursuivie afin de presser les parties osseuses l'une contre l'autre et d'assurer l'ancrage profond des extrémités des branches recourbées dans la paroi osseuse.

Le manchon est alors serti sur la tige, puis la portion de cette dernière qui dépasse au-delà du manchon est coupée.

Ce dispositif présente des dimensions relativement réduites et peut être utilisé sur tout type de site osseux, même de petites dimensions. Un jeu de plusieurs éléments en forme de grappin peut être prévu, chaque élément ayant des longueurs de tige et des longueurs de branches recourbées différentes, adaptées aux différents types d'ostéotomie ou de fractures pouvant se présenter.

Grâce à l'écartement des extrémités des branches recourbées, l'élément en forme de grappin a une bonne assise par rapport à l'os et est parfaitement ancré.

La forme de cet élément, alliée à la possibilité d'orientation des branches recourbées, permet l'adaptation de l'ancrage à la configuration particulière du site ou à la présence d'un tendon à proximité de la zone d'ancrage.

La tige centrale assure, quant à elle, un maintien semi-rigide des parties osseuses à réunir, avec une mise en tension de l'élément en forme de grappin. Cette semi-rigidité fait que le dispositif s'adapte à la relative élasticité naturelle de l'os, et peut être implanté dans des emplacements osseux subissant des contraintes en traction. La mise en tension de l'élément en forme de grappin permet le soudage des parties osseuses dans les meilleures conditions.

L'implantation de ce dispositif ne requiert aucune vis venant traverser les parois osseuses, et n'implique aucune détérioration du site.

De préférence, la tige centrale de l'élément en forme de grappin est en acier inoxydable ou en titane et a un diamètre de l'ordre de 1,2 millimètre, ce qui lui donne la semi-rigidité adéquate.

Avantageusement, un fil souple de traction est amarré à l'anneau de l'élément en forme de grappin. Ce fil permet de faciliter la traction de cet élément et le rapprochement des parties osseuses à maintenir.

L'instrument de mise en place du dispositif présente, quant à lui, un corps rigide comprenant un alésage longitudinal de diamètre supérieur à la largeur de l'anneau de la tige centrale mais inférieur à celui du manchon à sertir, et un tambour, solidaire de moyens de manoeuvre en rotation, situé en arrière de l'alésage et en communication avec lui.

Une fois l'élément en forme de grappin engagé au travers des parties osseuses, le fil souple de traction est introduit dans l'alésage de l'instrument puis est enroulé sur le tambour. L'instrument est ensuite engagé sur l'extrémité de l'élément en forme de grappin dépassant de l'os, jusqu'à venir en appui contre le manchon, et le tambour est manoeuvré en rotation de manière à exercer une traction sur le fil, et donc à rapprocher les parties osseuses et mettre le dispositif sous tension.

Pour sa bonne compréhension, l'invention est à nouveau décrite ci-dessous en référence au dessin schématique annexé représentant, à titre d'exemple non limitatif, une forme de réalisation préférée du dispositif qu'elle concerne.
La figure 1 en est une vue avant implantation sur une articulation du genou dont le plateau tibial est fracturé ;
les figures 2 à 4 en sont des vues similaires à la figure 1, au cours de trois phases successives d'implantation, et
les figures 5 et 6 sont des vues simplifiées de ce dispositif, après implantation sur l'extrémité d'un fémur.

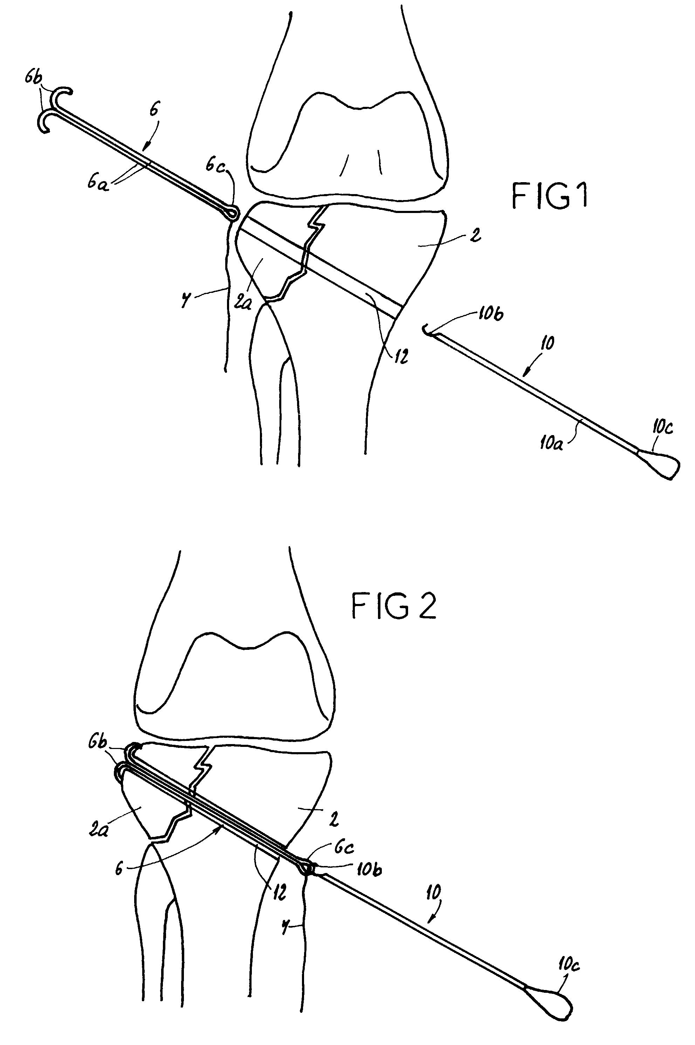

Les figures 1 à 4 représentent une articulation du genou, avec fracture au niveau du plateau tibial 2, et un dispositif de réduction de cette fracture et de maintien des parties fracturées 2,2a, une fois celles-ci réunies.

Le dispositif comprend un élément 6 en forme de grappin, formé par pliage adéquat d'une tige métallique, une rondelle d'arrêt 8 et un manchon métallique 9, apte à être serti sur l'élément 6.

L'élément 6 présente une tige centrale 6a, apte à traverser les parties osseuses 2,2a. Cette tige 6a est en acier inoydable et a un diamètre de 1,2 millimètre, de manière à être semi-rigide, c'est-à-dire à présenter une souplesse relative perpendiculairement à son axe longitudinal.

A une extrémité, la tige 6a est prolongée par deux branches 6b recourbées sur sensiblement 180 degrés, dont les extrémités libres sont acérées. A son autre extrémité, la tige 6a comprend un anneau 6c pour la traction de l'élément 6 à travers les parties osseuses 2,2a.

Un fil souple de traction 7 est amarré à l'anneau 6c.

Les figures 1 et 2 représentent également deux instruments 10,11 adaptés à la mise en place du dispositif.

L'instrument 10 comprend un corps allongé 10a, apte à être engagé dans le trou osseux 12 destiné à recevoir la tige 6a. A une extrémité, ce corps 10a présente une patte l0b, recourbée pour former un crochet pouvant être engagé dans l'anneau 6c. A son autre extrémité, le corps 10a est muni d'une poignée 10c de préhension, formée par une boucle métallique.

L'instrument 11 présente un corps rigide 11a, dans lequel est aménagé un évidement 11b, et qui est prolongé, à une extrémité, par une crosse 11c de préhension. A son autre extrémité, il comprend un alésage longitudinal 13, de diamètre supérieur à la largeur de l'anneau 6c mais inférieur à celui du manchon 9, débouchant dans l'évidement 11b par un trou coaxial de diamètre supérieur au diamètre du fil 7. A hauteur approximative du raccordement du corps 11a et de la crosse 11c, l'instrument 11 comprend également un tambour 14, solidaire d'une clef 15 de manoeuvre en rotation. Ce tambour 14 est situé en arrière et en face d'un trou, coaxial à l'alésage 13, de diamètre supérieur au diamètre du fil 7, qui débouche également dans l'évidement 11b.

Après aménagement du trou 12 dans les parties osseuses 2,2a, plus ou moins perpendiculairement à la fracture, l'instrument 10 est engagé dans ce trou 12 puis dans l'anneau 6c.

L'élément 6 est ensuite tiré dans le trou 12 jusqu'à ce que son extrémité pourvue de l'anneau 6c débouche au-delà de l'os, du côté opposé au côté d'introduction.

Le fil 7 est entièrement tiré au travers du trou 12, comme le montre la figure 2.

L'élément 6 peut, le cas échéant, être pivoté sur lui-même de manière à orienter ses branches recourbées 6b de la manière la plus appropriée en fonction de la configuration spécifique du site osseux ou de la présence éventuelle d'un ancrage ligamentaire.

La rondelle 8 et le manchon 9 sont engagés sur l'extrémité de la tige 6a dépassant de l'os, jusqu'à venue de la rondelle 8 en appui contre la paroi osseuse, puis le fil 7 est introduit dans l'alésage 13 et dans les trous consécutifs à celui-ci et est enroulé sur le tambour 14.

L'instrument 11 est ensuite engagé sur l'extrémité de l'élément 6 dépassant de l'os, jusqu'à venir en appui contre le manchon 9, et le tambour 14 est manoeuvré en rotation de manière à exercer une traction sur le fil 7, et donc sur la tige 6a.

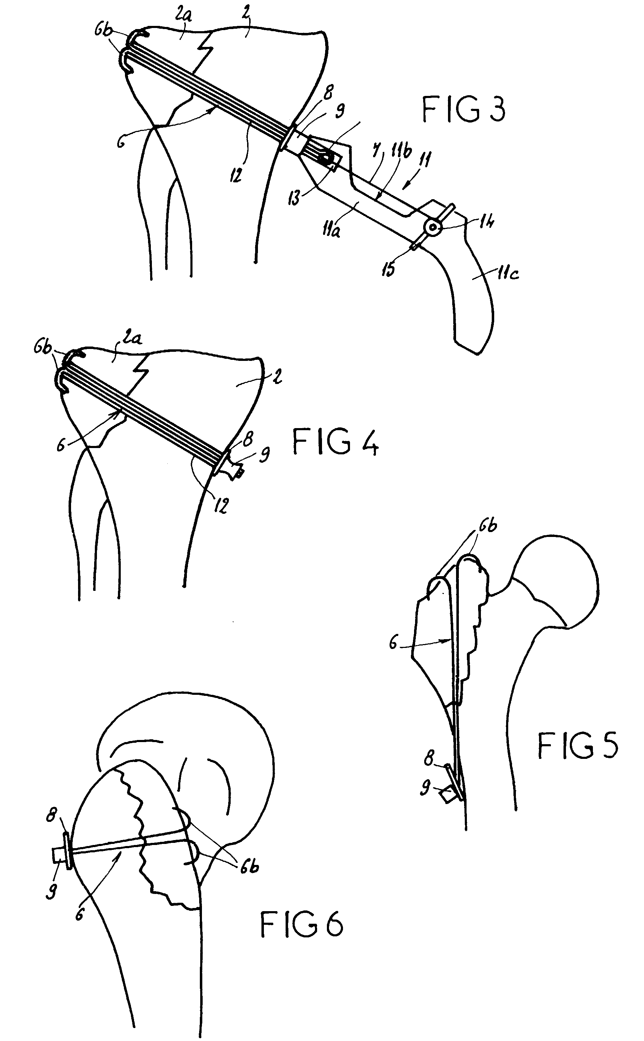

Cette traction permet d'insérer les extrémités des branches recourbées 6b dans la paroi osseuse puis de rapprocher progressivement les parties osseuses fracturées 2,2a, comme le montre la figure 3.

Une fois les parties osseuses 2,2a réunies, la traction est poursuivie afin de presser ces parties osseuses 2,2a l'une contre l'autre et d'assurer l'ancrage profond des extrémités des branches recourbées 6b.

Le manchon 9 est alors serti sur la tige 6a, puis la portion de cette dernière qui dépasse au-delà du manchon 9 est coupée, comme le montre la figure 4.

Le dispositif présente des dimensions relativement réduites et peut être utilisé sur tout type de site osseux, même de petites dimensions.

Les figures 5 et 6 montrent l'implantation du dispositif dans le grand trochanter. La tige 6a peut être pliée au niveau de son extrémité dépassant de l'os, si nécessaire pour l'appui de la rondelle 8.

Les branches recourbées 6b ont une souplesse relative leur permettant de s'adapter à la configuration particulière du site osseux, comme le montre la figure 5 dans le cas du grand trochanter, dans un cas toutefois extrême de déformation.

Le dispositif s'avère également très approprié en cas de fracture de la rotule ou de fractures de certaines parties d'articulations, comme l'olécrane.

Un jeu de plusieurs éléments 6 peut être prévu, chaque élément ayant des longueurs de tige 6a et des longueurs de branches 6b différentes, adaptées aux différents types de fractures pouvant se présenter. Par exemple, des éléments 6 ayant des longueurs totales de 10, 15, 18, 20, 100, ou 140 millimètres et des largeurs respectives au niveau des branches recourbées 6b de 15, 15, 18, 20, 15 ou 15 millimètres peuvent être envisagées.

Grâce à l'écartement des extrémités des branches recourbées 6b, l'élément 6 a une bonne assise par rapport à l'os et est parfaitement ancré.

La forme de cet élément 6, alliée à la possibilité d'orientation des branches 6b, permet l'adaptation de l'ancrage à la configuration particulière du site ou à la présence d'un tendon à proximité de la zone d'ancrage.

La tige centrale 6a assure, quant à elle, un maintien semi-rigide des parties osseuses 2,2a à réunir, avec une mise en tension, ce qui permet l'adaptation du dispositif à la relative élasticité naturelle de l'os et le soudage de ces parties dans les meilleures conditions.

L'implantation de ce dispositif ne requiert en outre aucune vis venant traverser les parois osseuses et n'implique aucune détérioration du site.

## Revendications

1. Dispositif de maintien de parties osseuses après ostéotomie, ou de réduction d'une fracture osseuse et de maintien des parties osseuses fracturées, une fois celles-ci réunies, caractérisé en ce qu'il comprend :
- un élément (6) en forme de grappin, comprenant une tige centrale (6a) semi-rigide, c'est-à-dire présentant une souplesse relative perpendiculairement à son axe longitudinal, apte à traverser les parties osseuses (2,2a) à maintenir, cette tige (6a) étant prolongée, à une extrémité, par au moins deux branches (6b), recourbées sur sensiblement 180 degrés, dont les extrémités libres sont acérées, et comprenant, à son autre extrémité, un anneau (6c) pour la traction de l'élément en forme de grappin (6) à travers les parties osseuses (2,2a) à maintenir ;
- une rondelle d'arrêt (8), destinée à être engagée sur la tige (6a), après que celle-ci ait traversé les parties osseuses (2,2a) à maintenir, et à venir en appui contre l'une de ces parties osseuses (2,2a), et
- un manchon métallique (9), apte à être engagé sur la tige (6a) jusqu'à venir en appui contre la rondelle d'arrêt (9), et à être serti sur la tige (6a) après réunion des parties osseuses à maintenir et mise en tension de l'élément (6) en forme de grappin.

2. Dispositif selon la revendication 1, caractérisé en ce que la tige centrale (6a) de l'élément (6) en forme de grappin est en acier inoxydable ou en titane et a un diamètre de l'ordre de 1,2 millimètre.

3. Dispositif selon la revendication 1 ou la revendication 2, caractérisé en ce qu'un fil souple (7) de traction est amarré à l'anneau (6c) de l'élément (6) en forme de grappin.

4. Dispositif selon l'une des revendications 1 à 3, caractérisé en ce qu'il comprend un jeu de plusieurs éléments (6) en forme de grappin, chaque élément (6) ayant des longueurs de tige (6a) et des longueurs de branches recourbées (6b) différentes, adaptées aux différents types de fractures pouvant se présenter.

5. Instrument de mise en place du dispositif selon la revendication 3 ou la revendication 4, caractérisé en ce qu'il présente un corps rigide (11a) comprenant un alésage longitudinal (13) de diamètre supérieur à la largeur de l'anneau (6c) de la tige centrale (6a) mais inférieur à celui du manchon (9) à sertir, et un tambour (14), solidaire de moyens de manoeuvre en rotation (15), situé en arrière de l'alésage (13) et en communication avec lui.
